# EUROPEAN PATENT SPECIFICATION

(11) **EP 4 218 844 B1**
(45) Date of publication and mention of the grant of the patent: **18.11.2026**
(21) Application number: 22216541.7
(22) Date of filing: 11.01.2019
(51) Int. Cl.: A61L 31/04, A61L 31/16, A61L 26/00, A61K 31/722

(54) **POWDER FOR ACHIEVING HEMOSTASIS**
PULVER FÜR HÄMOSTASE
DISPOSITIF POUR RÉALISER UNE HÉMOSTASE

(30) Priority: 12.01.2018 US 201862616751 P
(43) Date of publication of application: 02.08.2023
(62) Document number(s) of the earlier application(s) in accordance with Art. 76 EPC: 19703448.1 / 3 737 432
(73) Proprietor: Boston Scientific Scimed, Inc., Maple Grove, MN 55311-1566 (US)
(72) Inventor: FREDRICKSON, Gerald, Westford, MA 01886 (US); SMITH, Amanda L., Boston, MA 02115 (US); PIC, Andrew, Northborough, MA 01532 (US); GERVASIO, Sophia, Coventry, RI 02816 (US); LYDECKER, Lauren, Millbury, MA 01527 (US)
(74) Representative: Vossius & Partner Patentanwälte Rechtsanwälte mbB

(56) References cited:
- EP-A1- 3 081 236
- WO-A1-2007/074327
- WO-A1-2009/028965
- WO-A1-2012/058312
- WO-A1-2014/191738
- WO-A1-2016/109847
- WO-A2-2009/132224
- WO-A2-2013/053753
- US-A1- 2004 101 546
- US-A1- 2004 265 371
- DATABASE WPI Week 201214, Derwent World Patents Index; AN 2011-Q77774, XP002790475
- ISHAQ SAUID ET AL: "Use of hemostatic powder in bleeding portal hypertensive gastropathy", vol. 2, no. 9, 30 September 2017 (2017-09-30), pages 238 - 240, XP093050747, Retrieved from the Internet <URL:https://www.videogie.org/action/showPdf?pii=S2468-4481%2817%2930114-5> DOI: https://doi.org/10.1016/j.vgie.2017.06.004

## Description

### CROSS-REFERENCE TO RELATED APPLICATIONS

This application claims the benefit of U.S. Provisional Application No. 62/616,751, filed January 12, 2018, entitled "Powder for Achieving Hemostasis."

### BACKGROUND

Gastrointestinal bleeding affects millions of people annually. Certain cases of internal bleeding cannot be controlled effectively by current hemostatic technologies such as clips, cautery, or band ligation. Wounds, surgical sites, diseased tissue, ulcer beds and gastric varices, among others, are locations where conventional means of hemostasis may to fail, leading to extended hospital stay or death.

WO 2012/058312 A1 discloses wound sealing compositions comprising dry powdered mixtures of chitosan particles and oxidised polysaccharide particles.

### SUMMARY

In various aspects, the present disclosure pertains compositions for use in methods of treating or preventing bleeding at a tissue site comprising applying a powder composition as defined in claim 1.

In various embodiments, the tissue site may be a body lumen, for example a site in the gastrointestinal tract. When the tissue site is a body lumen, the chitosan powder may be applied, for example, via a catheter or other suitable device.

In various embodiments, which may be used in conjunction with the above aspects and embodiments, the powder may be fluidized in a gas (e.g., CO₂, nitrogen, air, etc.) to form a fluidized powder and blown onto the tissue site. In such embodiments, the fluidized powder may exit the catheter at a velocity ranging from 15 to 50 m/s, among other possible velocities.

In various aspects, the present disclosure pertains to powder compositions for application to a tissue site, where the powder composition comprising aldehyde modified chitosan derivatized by reacting a multifunctional aldehyde with amine groups on chitosan admixed with second particles that comprise a crosslinking agent that covalently or non-covalently interacts with the first particles upon exposure to moisture.

In some embodiments, the derivatized chitosan reacts with cysteine groups in tissue upon exposure to moisture.

In some embodiments, which may be used in conjunction with the above aspects and embodiments, the derivatized chitosan may interact with thiol groups in tissue upon exposure to moisture.

In various aspects, the present disclosure pertains to the powder compositions for application to a tissue site that comprise a chitosan salt.

In some embodiments, the chitosan salt ionically crosslinks with negative charged species in tissue or blood.

In various aspects, which may be used in conjunction with the above aspects and embodiments, the disclosure pertains to catheter assemblies, which are preloaded with the chitosan powder composition and which are configured to deliver the chitosan powder composition a tissue site.

These and other aspects and embodiments are further described in the detailed description to follow.

### DETAILED DESCRIPTION

In various aspects, the present disclosure pertains to compositions for use in methods of treating a tissue site (e.g., a wound, a surgical site, a diseased tissue site, an ulcer bed, a gastric varix, etc.), in which a chitosan powder is applied to the tissue site. The chitosan powder may be applied, for example, to address existing bleeding or to prevent or minimize future bleeding that may occur. In various embodiments, the tissue site is tissue that surrounds a body lumen, for example, a wall of the gastrointestinal tract.

In various embodiments, the chitosan powder may be applied to a tissue site via a catheter. Examples include catheter assemblies in which a powder may be fluidized in a gas (e.g., compressed air, nitrogen, carbon dioxide, etc.) to form fluidized powder, which is then blown onto the tissue site. For example, a catheter assembly may be provided, which includes (a) a catheter having a lumen extending therethrough, a proximal end, and a distal end having an exit orifice, and (b) a reservoir containing a chitosan powder. The catheter assembly may be configured to deliver the chitosan powder from the reservoir, through the lumen, and out the exit orifice. In certain embodiments, the catheter assembly may include a pressurized reservoir that contains a pressurized gas for delivering the chitosan powder from the reservoir, through the lumen, and out the exit orifice. For example, the pressurized reservoir may be positioned upstream of the reservoir and the pressurized gas passed through the chitosan powder, thereby fluidizing the chitosan powder in the gas for delivery of through the lumen and out the exit orifice. In certain embodiments, the catheter is operated such that the fluidized powder exits the catheter at a velocity ranging from 15 m/s to 50 m/s. Where applied to the gastrointestinal tract, the chitosan powder may be applied through an endoscope.

In various aspects, the present disclosure pertains to catheters that are preloaded with the chitosan powder. For example, a system may be provided, which includes a catheter having a proximal end and a distal end that is partially loaded with chitosan powder (with a remaining volume being air). The catheter may also include a seal, such as a plug, cap, or other mechanism for retaining the chitosan powder on the proximal and distal ends of the catheter. The system may further be provided with a mechanism for breaking the proximal seal and for applying gas to the catheter at sufficient pressure to fluidize the powder in the catheter, eject the distal seal, and disperse the fluidized chitosan powder from the distal end of the catheter and onto the treatment site.

When applied to a tissue site, chitosan powder in accordance with the present disclosure acts as a barrier to bleeding associated with the tissue site. The chitosan powder acts as a barrier by absorbing liquids, which, for example, may be bodily fluids such as blood or gastrointestinal fluids (e.g., pancreatic juices, biliary fluid, saliva, etc.) that are present at the tissue site, or may be fluid such as saline, phosphate buffered saline, or contrast fluid that is applied to the tissue site prior to, concurrently with, or subsequent to application of the chitosan powder. The chitosan powder may be used to achieve hemostasis at sites of active bleeding or can be used as a preventative over clipped areas, suture sites, or other having the potential for bleeding, among other uses.

In various aspects, the present disclosure pertains to chitosan powders, that may be used to for hemostasis, among other possible uses.

Chitosan powders for use in the present disclosure may be of any suitable particle size. In various embodiments, the particle size may range, for example, from less than 1 µm to 1000 µm (e.g., ranging from 1 µm to 2.5 µm to 5 µm to 10 µm to 25 µm to 50 µm to 100 µm to 250 µm to 500 µm to 1000 µm), among other possibilities. In this regard, a chitosan powder having particles sized between 50 µm and 425 µm performs well when dispensed through an 8 French catheter.

As noted above, chitosan powder may contain, a chitosan salt, and, optionally, a natural or synthetic polymer.

Chitosan is a modified polysaccharide containing randomly distributed β-(1-4)-linked D-glucosamine and N-acetyl-D-glucosamine monomer units. Chitosan is produced commercially by the alkaline N-deacetylation of chitin, which is a cellulose-like polymer consisting primarily of unbranched chains of modified glucose, specifically N-acetyl-D-glucosamine.

The degree of deacetylation in commercial chitosans typically ranges from 75 to 100% although essentially any degree of deacetylation is possible. Chitosan is positively charged in acidic to neutral solutions with a charge density that is dependent on the pH and the degree of deacetylation. The pka value of chitosan generally ranges from 6.1 to 7.0, depending on the degree of deacetylation. Thus, while typically substantially insoluble in distilled water, chitosan is generally soluble in aqueous acidic solutions (e.g., pH ^{~}6.5 or less).

Examples of chitosan salts include chitosan halides such as chitosan fluoride, chitosan chloride, chitosan bromide, chitosan iodide, chitosan phosphate, chitosan nitrate, chitosan sulfate, chitosan salts of organic mono-acids such as formate, acetate, propionate, butyrate, chitosan salts of organic diacids such as oxalate, malonate, succinate, maleate, or glutarate, or salts of hydroxyacids such as glycolate, lactate, tartrate, malate, citrate, or gluconate.

In various embodiments, modified chitosans may be employed, which exhibit enhanced properties, including enhanced adhesion.

The modified chitosans include chitosan modified with groups that allow for covalent reaction with tissue, including groups that are reactive with amine groups found in tissue. The multifunctional (e.g., difunctional, trifunctional, etc.) reactive molecule which is a multifunctional aldehyde molecule is reacted with amine groups on chitosan to form aldehyde-modified chitosan (chitosan having pendant aldehyde groups). **In** certain specific embodiments, the multifunctional reactive molecule **may be reacted with** chitosan in relative amounts such that multifunctional reactive molecule is provided in a 1x molar minimum relative to the number of moles of amine groups on the chitosan, such that all or essentially all of the amine groups are reacted and have pendant reactive groups.

Example of multifunctional aldehydes include glutaraldehyde, glyoxal, and aldehyde terminated hydrophilic polymers. Example of multifunctional epoxides include 4-butanediol diglycidyl ether, ethylene glycol diglycidyl ether, and epoxide terminated hydrophilic polymers. Hydrophilic polymers which may be provided with aldehyde or epoxide termination include poly(ethylene glycol) (PEG), also referred to as poly(ethylene oxide) (PEO), polyvinyl alcohol (PVA), polyvinyl pyrrolidone (PVP), polyacrylamide, poly(acrylic acid), and poly(hydroxyethyl methacrylate) (PHEMA). Suitable hydrophilic polymers may range, for example, from 2 to 250 monomers in length, among other possibilities.

In certain specific embodiments, a modified chitosan may be formed by reacting a reactive synthetic molecule such as a PEG dialdehyde with chitosan in relative amounts such that the reactive molecule is provided in a 1x molar minimum relative to the number of moles of amine groups on the chitosan, such that all or essentially all of the amine groups of the chitosan are reacted and have pendant aldehyde-terminated PEG groups.

In some embodiments, chitosan or modified chitosan may be covalently crosslinked prior to administration, and subsequently applied to tissue. For instance, chitosan or modified chitosan may be reacted with a multifunctional molecule having two or more groups (i.e. aldehyde groups) that are reactive with the chitosan (e.g., reactive with the amine groups on the chitosan or carboxymethyl groups on the modified chitosan). The resulting product is subsequently applied to tissue, after grinding the product into a powder, if desired or necessary. This should generally improve the overall structural integrity of the powder.

In some embodiments, a chitosan powder is provided, which become covalently crosslinked upon administration to tissue.

### EXAMPLE (not according to the claims)

Chitosan obtained from Sigma Aldrich is suspended in water at a concentration of 2 wt% Chitosan and 98 wt% water. The mixture is stirred using a mechanical mixer at room temperature. Acetic acid is then added during the stirring such that the pH levels out near 5.0 after 5 hours of stirring. 2 wt% citric acid (relative to the weight of chitosan initially used) is added to the container and mixed for an additional 5 hours. This process forms a gel which is subsequently dried. The dried gel is then ground into a fine powder for use.

## Claims

1. A powder composition for application to a tissue site, the powder composition comprising aldehyde modified chitosan derivatized by reacting a multifunctional aldehyde with amine groups on chitosan.

2. The powder composition of claim 1, comprising a first powder comprising the chitosan derivatized with a multifunctional aldehyde admixed with chitosan or a chitosan salt powder.

3. The powder composition of claim 1 or 2, wherein the chitosan derivatized with the multifunctional aldehyde comprises pendant aldehyde groups.

4. The powder composition of claim 1 or 2, wherein the multifunctional aldehyde is a di- or trifunctional aldehyde.

5. The powder composition of any of claims 1 to 3, wherein the multifunctional aldehyde comprises glutaraldehyde, glyoxal, or an aldehyde terminated hydrophilic polymer.

6. The powder composition of claim 5, wherein the aldehyde terminated hydrophilic polymer comprises poly(ethylene glycol), polyvinyl alcohol, polyvinyl pyrrolidone, polyacrylamide, poly(acrylic acid), or poly(hydroxyethyl methacrylate.

7. The powder composition of any of claims 1 to 3, wherein the multifunctional aldehyde is poly(ethylene glycol) dialdehyde.

8. The powder composition of any one of claims 1 to 7 for use in treating or preventing bleeding at a tissue site in the gastrointestinal tract.

9. The powder composition for use of claim 8, wherein the composition is applied to the tissue site via a catheter.

10. The powder composition for use of claim 8 or 9, wherein the composition is fluidized in a gas to form a fluidized powder and blown onto the tissue site.

11. The powder composition for use of claim 10, wherein the gas is CO₂.

12. The powder composition for use of claim 10 or 11, wherein the fluidized powder is applied using a catheter, and wherein the fluidized powder exits the catheter at a velocity ranging from 15 m/s to 50 m/s.

13. The powder composition for use of any one of claims 8 to 12, wherein the composition is applied to the gastrointestinal tract through an endoscope.

## Patentansprüche

1. Eine Pulverzusammensetzung zum Aufbringen auf eine Gewebestelle, wobei die Pulverzusammensetzung mit Aldehyd modifiziertes Chitosan umfasst, das durch Umsetzen eines multifunktionellen Aldehyds mit Amingruppen an Chitosan derivatisiert ist.

2. Die Pulverzusammensetzung nach Anspruch 1, umfassend ein erstes Pulver, welches das mit einem multifunktionellen Aldehyd derivatisierte Chitosan im Gemisch mit Chitosan oder einem Chitosansalzpulver umfasst.

3. Die Pulverzusammensetzung nach Anspruch 1 oder 2, wobei das mit dem multifunktionellen Aldehyd derivatisierte Chitosan seitenständige Aldehydgruppen umfasst.

4. Die Pulverzusammensetzung nach Anspruch 1 oder 2, wobei das multifunktionelle Aldehyd ein di- oder trifunktionelles Aldehyd ist.

5. Die Pulverzusammensetzung nach einem der Ansprüche 1 bis 3, wobei das multifunktionelle Aldehyd Glutaraldeyhd, Glyoxal oder ein Aldehyd-terminiertes hydrophiles Polymer umfasst.

6. Die Pulverzusammensetzung nach Anspruch 5, wobei das Aldehyd-terminierte hydrophile Polymer Aldehyd Poly(ethylenglykol), Polyvinylalkohol, Polyvinylpyrrolidon, Polyacrylamid, Poly(acrylsäure) oder Poly(hydroxyethylmethacrylat) umfasst.

7. Die Pulverzusammensetzung nach einem der Ansprüche 1 bis 3, wobei das multifunktionelle Aldehyd Poly(ethylenglykol)dialdehyd ist.

8. Die Pulverzusammensetzung nach einem der Ansprüche 1 bis 7 zur Verwendung bei der Behandlung oder Vorbeugung von Blutungen an einer Gewebestelle im Magen-Darm-Trakt.

9. Die Pulverzusammensetzung zur Verwendung nach Anspruch 8, wobei die Zusammensetzung über einen Katheter auf die Gewebestelle aufgebracht wird.

10. Die Pulverzusammensetzung zur Verwendung nach Anspruch 8 oder 9, wobei die Zusammensetzung in einem Gas fluidisiert ist, um ein fluidisiertes Pulver zu bilden, und auf die Gewebestelle geblasen wird.

11. Die Pulverzusammensetzung zur Verwendung nach Anspruch 10, wobei das Gas CO₂ ist.

12. Die Pulverzusammensetzung zur Verwendung nach Anspruch 10 oder 11, wobei das fluidisierte Pulver unter Verwendung eines Katheters aufgebracht wird und wobei das fluidisierte Pulver aus dem Katheter mit einer Geschwindigkeit im Bereich von 15 m/s bis 50 m/s austritt.

13. Die Pulverzusammensetzung zur Verwendung nach einem der Ansprüche 8 bis 12, wobei die Zusammensetzung durch ein Endoskop in den Magen-Darm-Trakt verabreicht wird.

## Revendications

1. Composition en poudre pour l'application à un site tissulaire, la composition en poudre comprenant un chitosane modifié par un aldéhyde transformé en dérivé par réaction d'un aldéhyde multifonctionnel avec des groupes amine sur le chitosane.

2. Composition en poudre selon la revendication 1, comprenant une première poudre comprenant le chitosane transformé en dérivé avec un aldéhyde multifonctionnel mélangé avec du chitosane ou une poudre de sel de chitosane.

3. Composition en poudre selon la revendication 1 ou 2, dans laquelle le chitosane transformé en dérivé avec un aldéhyde multifonctionnel comprend des groupes aldéhyde pendants.

4. Composition en poudre selon la revendication 1 ou 2, dans laquelle l'aldéhyde multifonctionnel est un aldéhyde di- ou tri-fonctionnel.

5. Composition en poudre selon l'une quelconque des revendications 1 à 3, dans laquelle l'aldéhyde multifonctionnel comprend du glutaraldéhyde, du glyoxal, ou un polymère hydrophile terminé par un aldéhyde.

6. Composition en poudre selon la revendication 5, dans laquelle le polymère hydrophile terminé par un aldéhyde comprend du polyéthylèneglycol, du poly(alcool vinylique), de la polyvinylpyrrolidone, du polyacrylamide, du poly(acide acrylique), ou du poly(méthacrylate d'hydroxyéthyle).

7. Composition en poudre selon l'une quelconque des revendications 1 à 3, dans laquelle l'aldéhyde multifonctionnel est le dialdéhyde de polyéthylèneglycol.

8. Composition en poudre selon l'une quelconque des revendications 1 à 7, pour une utilisation dans le traitement ou la prévention du saignement au niveau d'un site tissulaire dans le tractus gastro-intestinal.

9. Composition en poudre pour une utilisation selon la revendication 8, dans laquelle la composition est appliquée au site tissulaire via un cathéter.

10. Composition en poudre pour une utilisation selon la revendication 8 ou 9, dans laquelle la composition est fluidisée dans un gaz pour former une poudre fluidisée et est soufflée sur le site tissulaire.

11. Composition en poudre pour une utilisation selon la revendication 10, dans laquelle le gaz est CO₂.

12. Composition en poudre pour une utilisation selon la revendication 10 ou 11, dans laquelle la poudre fluidisée est appliquée par le biais d'un cathéter, et dans laquelle la poudre fluidisée sort du cathéter à une vitesse située dans la plage allant de 15 m/s à 50 m/s.

13. Composition en poudre pour une utilisation selon l'une quelconque des revendications 8 à 12, dans laquelle la composition est appliquée au tractus gastro-intestinal par le biais d'un endoscope.
